# EUROPEAN PATENT APPLICATION

(11) **EP 4 618 095 A1**
(43) Date of publication of application: **17.09.2025**
(21) Application number: 24163940.0
(22) Date of filing: 15.03.2024
(51) Int. Cl.: G16H 20/70, G16H 40/63, A61B 5/16, A61M 21/00, G16H 80/00, G16H 40/67

(54) **RANDOM INTERVAL REPETITION TESTING APPARATUS AND METHOD FOR CONTROLLING SAME**

(71) Applicant: Moovd Holding B.V., 7442 DA Nijverdal (NL)
(72) Inventor: THEUNISSEN, Tjeu Petrus Maria, NIJVERDAL (NL); NOUR, Mohamad, NIJVERDAL (NL); KAMPHUIS, Gerrit Jan Sander, NIJVERDAL (NL)
(74) Representative: Algemeen Octrooi- en Merkenbureau B.V.

(57) **Abstract**

A computer-implemented method of controlling a Random Interval Repetition, RIR, testing apparatus; the method comprising: monitoring a stimulus presented on an output interface of the RIR testing apparatus; monitoring a reaction input to the RIR testing apparatus in reaction to the stimulus; based on at least one of: a reaction time of the reaction input; and a correctness of the reaction input, changing at least one of: an adaptation rate of the stimulus; and an allowable reaction time during which a subsequent reaction to the stimulus should be input; and repeating the method at least once.

## Description

### TECHNICAL FIELD

The present disclosure generally relates to RIR testing. Particular embodiments relate to a computer-implemented method of controlling a Random Interval Repetition, RIR, testing apparatus, a computer program, a computer-readable storage medium, a data processing apparatus, and a RIR testing apparatus.

### BACKGROUND

Random Interval Repetition (RIR) testing involves its application as a method for assessing the impact of various cognitive tasks on working memory (WM) resources. RIR testing is specifically designed to measure the cognitive load imposed by additional tasks on WM capacity.

In RIR testing, participating test subjects are presented with a primary task, such as responding to a stimulus, while concurrently engaging in a secondary task that is meant to tax WM resources, such as making eye movements or performing mental arithmetic. Test subjects are required to respond as quickly as possible to the primary task while juggling the secondary task, providing insight into the cognitive demands placed on WM.

The primary outcome measure in RIR testing is typically the difference in reaction time between conditions with and without the secondary task. This provides a quantifiable indicator of the additional WM load imposed by the secondary task.

RIR testing has been applied across various domains, including psychology, neuroscience, and cognitive science, to investigate how different cognitive tasks impact WM capacity. This versatility allows researchers to explore a wide range of cognitive processes and phenomena.

For example, RIR testing can be used in Eye Movement Desensitization and Reprocessing (EMDR) therapy, which involves focusing on traumatic memories in a manner similar to exposure therapy while engaging in tasks that load WM, e.g. side-to-side eye movements or other forms of bilateral stimulation.

### SUMMARY

Various known RIR testing approaches take a long time, which may lead to accidental additional stress for the test subject, which is of course undesirable in contexts such as EMDR therapy.

It is therefore an aim of various embodiments according to the present disclosure to speed up RIR testing.

Accordingly, there is provided in a first aspect of the present disclosure, a computer-implemented method of controlling a Random Interval Repetition, RIR, testing apparatus; the method comprising:
- monitoring a stimulus presented on an output interface of the RIR testing apparatus;
- monitoring a reaction input to the RIR testing apparatus in reaction to the stimulus;
- based on at least one of: a reaction time of the reaction input; and a correctness of the reaction input, changing at least one of: an adaptation rate of the stimulus; and an allowable reaction time during which a subsequent reaction to the stimulus should be input;
and repeating the method at least once.

It is noted that, in this context, the term adaptation may be taken to refer to a varying nature of the stimulus, such as a movement speed, while the term change may be taken to refer to changing such a varying nature of the stimulus, e.g. changing that movement speed, and the term altering may be taken to refer to altering a way that the stimulus can be directly perceived.

The adaptation may be interpreted as adapting a salience of the stimulus, wherein the salience of the stimulus is defined as how easily perceivable the stimulus is to a test subject of the RIR testing apparatus.

In various embodiments, the adaptation rate of the stimulus comprises a visual movement speed if the stimulus comprises a visual stimulus, and/or the adaptation rate of the stimulus comprises an adaptation of a ratio of background noise to stimulus signal if the stimulus comprises an auditory stimulus.

In various embodiments, the method comprises: altering the stimulus, in order to elicit the reaction input in response to the alteration of the stimulus.

In various embodiments, said step of altering the stimulus is iterated over multiple iterations of the repeated method with a waiting time between iterations, the waiting time being selected randomly in a range of 0.5 to 2 seconds.

In a further developed embodiment, instead of including a waiting time, the method may comprise temporarily suppressing the stimulus after the reaction is input, wherein the temporary suppressing preferably spans a random time duration, preferably a random time duration between 0.5 and 2 seconds.

Preferably, the suppressing may comprise immobilizing the stimulus.

In various embodiments, said step of altering the stimulus comprises altering a visual shape of the stimulus if the stimulus comprises a visual stimulus, and/or altering a pitch tone of the stimulus if the stimulus comprises an auditory stimulus.

In various embodiments, the visual shape may preferably be a geometric shape defining a direction, such as an arrow. This has the benefit that most user input interfaces comprise arrow keys (physical or virtual) or direction pads, allowing a straightforward mapping.

Preferably, the test subject may be expected to input directions corresponding with the ascending or descending direction of the pitch tone. For example, the test subject may use the up and down keys for this task, as these auditory 'directions' can be intuitively mapped to (physical or virtual) common user interface keys or directional pads. Alternatively, the auditory stimulus may comprise a shift in left-right balance, which can be intuitively mapped to (physical or virtual) common user interface keys or directional pads.

Alternatively, the stimulus may comprise multiple pitch tones, played concurrently or subsequently.

In various embodiments, said step of changing the adaptation rate of the stimulus based on the correctness of the reaction input comprises:
- determining, over multiple iterations of the repeated method, a ratio of correct reaction inputs to total reaction inputs; and
- changing the adaptation rate proportionately to the ratio of correct reaction inputs to total reaction inputs.

In various embodiments, said step of changing the adaptation rate of the stimulus comprises changing the adaptation rate proportionately to a pre-determined maximum adaptation rate, and subject to a dynamic maximum adaptation rate defined as 1-(w^{T}), in which w is a weighting factor and T is the total reaction inputs.

In various embodiments, reaction inputs older than a pre-determined threshold, or reaction inputs that have been the longest in a queue threatening to overflow, are discarded.

In various embodiments, the allowable reaction time is based on an average reaction time of reaction inputs in previous iterations of the repeated method, and is preferably selected randomly within one standard deviation from said average reaction time.

In various embodiments, if the reaction input was correct:
- the allowable reaction time, during which the subsequent reaction to the stimulus should be input, is decreased; and/or
- the adaptation rate of the stimulus is increased, preferably up to a or the pre-determined maximum adaptation rate.

Additionally, there is provided in a second aspect of the present disclosure, a computer program comprising instructions which, when the program is executed by a computer, cause the computer to carry out the steps of the method of any one of the above-described method embodiments.

Additionally, there is provided in a third aspect of the present disclosure, a computer-readable storage medium comprising instructions which, when executed by a computer, cause the computer to carry out the steps of the method of any one of the above-described method embodiments.

Additionally, there is provided in a fourth aspect of the present disclosure, a data processing apparatus comprising means for carrying out the steps of the method of any one of the above-described method embodiments.

In various embodiments, the data processing apparatus may form a Random Interval Repetition, RIR, testing apparatus and may comprise: at least one processor; at least one memory; at least one stimulus output interface, preferably comprising a visual display and/or a sound speaker, arranged for outputting the stimulus to a test subject; and at least one reaction input interface arranged for detecting the reaction input from the test subject in response to the stimulus; the at least one memory storing instructions configured for, when executed by the at least one processor, controlling the RIR testing apparatus to carry out the steps of the method of any one of the above-described method embodiments.

In various embodiments, the at least one stimulus output interface comprises a vibration unit adapted to output a tactile stimulus.

Preferably, if various different types of stimulus, e.g. both a visual stimulus and an auditory stimulus, or both a visual stimulus and a tactile stimulus, or both an auditory stimulus and a tactile stimulus, or both a visual stimulus and an auditory stimulus and a tactile stimulus are combined, in a first exemplary embodiment the different types of stimulus may be synchronized and aligned so as to be interchangeable, whereas in an alternative second exemplary embodiment the different types of stimulus may require independent reactions regardless of whether or not the stimuli are synchronized.

The embodiments described herein are provided for illustrative purposes and should not be construed as limiting the scope of the invention. It is to be understood that the invention encompasses other embodiments and variations that are within the scope of the appended claims. The invention is not restricted to the specific configurations, arrangements, and features described herein. The invention has wide applicability and should not be limited to the specific examples provided. The embodiments disclosed are merely exemplary, and the skilled person will appreciate that various modifications and alternative designs can be made without departing from the scope of the invention.

### BRIEF DESCRIPTION OF THE DRAWINGS

In the following description, a number of exemplary embodiments will be described in more detail, to help understanding, with reference to the appended drawings, in which:
Figure 1 schematically illustrates a flowchart showing a first embodiment of the method according to the present disclosure;
Figure 2 schematically illustrates a flowchart showing a second embodiment of the method according to the present disclosure;
Figures 3A and 3B schematically illustrate several graphs showing execution of various embodiments of the method according to the present disclosure;
Figure 4 schematically illustrates an exemplary embodiment of the RIR testing apparatus according to the present disclosure; and
Figure 5 schematically illustrates a method operation loop of the stimulus when an exemplary embodiment of the method according to the present disclosure is performed.

### DETAILED DESCRIPTION

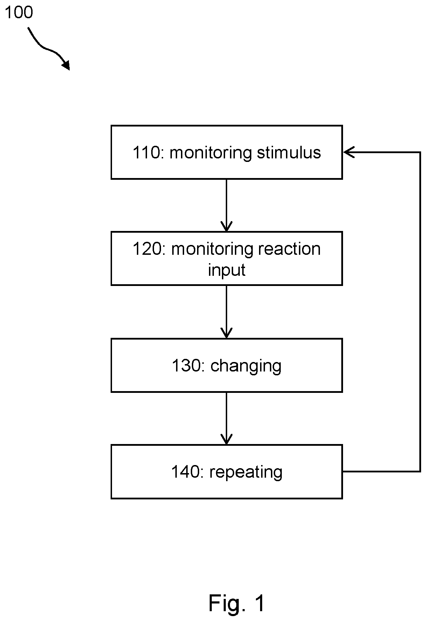

Figure 1 schematically illustrates a flowchart showing a first embodiment 100 of the method according to the present disclosure.

In step 110, a stimulus that is presented on an output interface of the RIR testing apparatus is monitored.

In step 120, a reaction that is input to the RIR testing apparatus in reaction to the stimulus is monitored.

In step 130, based on at least one of: a reaction time of the reaction input; and a correctness of the reaction input, at least one is changed of: an adaptation rate of the stimulus; and an allowable reaction time during which a subsequent reaction to the stimulus should be input.

In step 140, the method is repeated, at least once.

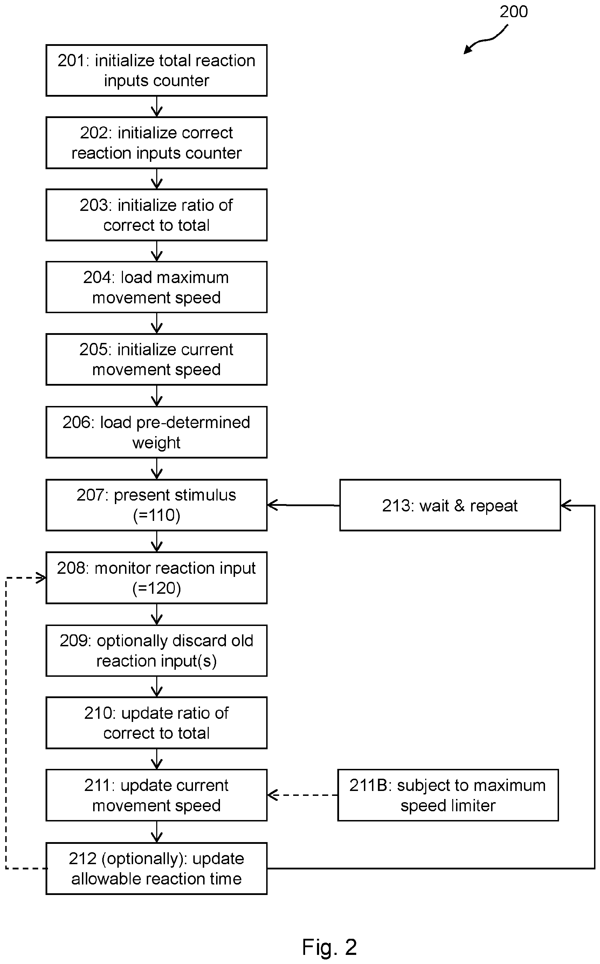

Figure 2 schematically illustrates a flowchart showing a second embodiment 200 of the method according to the present disclosure.

In step 201, a total reaction inputs counter may be initialized, which may keep track of a total number of reaction inputs that have so far been input by the test subject. It is noted that the full history of previous reaction inputs may be maintained, or that optionally old reaction inputs may be discarded (see step 209 below).

In step 202, a correct reaction inputs counter may be initialized, which may keep track of a number of correct reaction inputs (i.e. reaction inputs matching the stimulus) have so far been input by the test subject.

In step 203, a ratio of correct reaction inputs to total reaction inputs may be determined, on the basis of the counters resulting from steps 201 and 202. If the method is being initialized, a suitable starting point may be arbitrarily chosen, e.g. 100% or 80%.

In step 204, a maximum movement speed may be loaded.

In step 205, a current movement speed may be initialized, preferably to a sufficiently low value so as not to brusquely surprize the test subject.

In step 206, a pre-determined weight may be loaded.

In step 207, the stimulus may be presented, e.g. a visual shape may be shown on a visual output interface, such as a display or screen, to the test subject.

In step 208, the reaction input may be monitored, which may involve waiting (also see step 213 below) until a test subject's input triggers an analysis of said input.

In step 209, old reaction inputs may optionally be discarded. Of course, this step may instead (or also) be performed at a different point of the method execution, or may form part of a separate control routine altogether.

In step 210, the ratio of correct reaction inputs to total reaction inputs may be updated, now taking into account the reaction input monitored in step 208.

In step 211, the current movement speed may be updated, taking into account the ratio resulting from step 210.

In step 211B, the current movement speed may be subjected to a check or replacement in order to limit the current movement speed to a preferably dynamic maximum speed.

In step 212, the allowable reaction time for a subsequent reaction may optionally be updated.

In step 213, the method may involve a waiting time, and may be repeated, e.g. by returning to step 207.

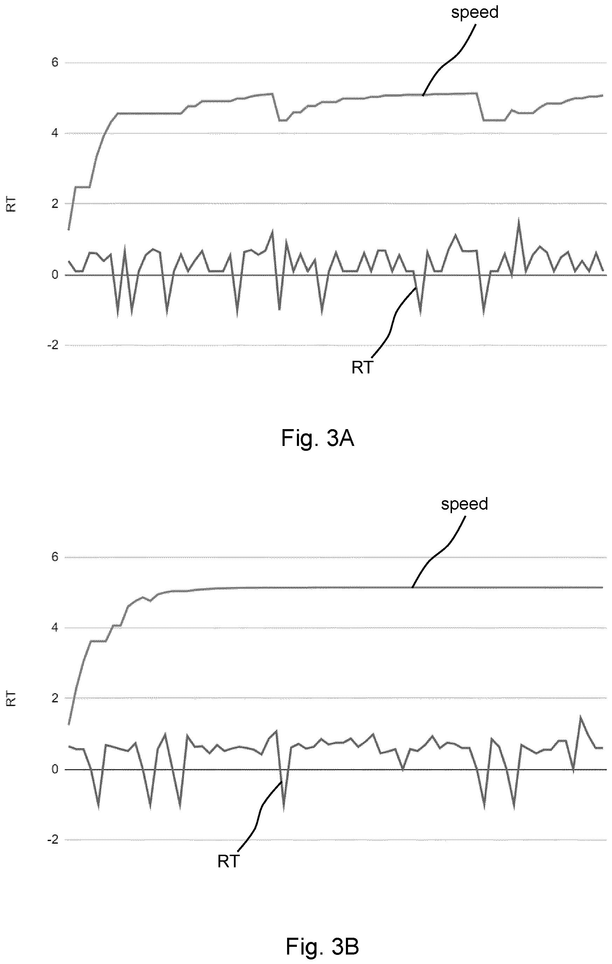

Figures 3A and 3B schematically illustrate several graphs showing execution of various embodiments of the method according to the present disclosure.

Fig. 3A schematically illustrates a exemplary plot of on the one hand (in the bottom region of the plot, near the horizontal axis) a varying reaction time (RT, on the vertical axis) as a function of time (on the horizontal axis), i.e. over multiple iterations of the exemplary embodiment of the method, and on the other hand (in the top region of the plot, starting from near 2 on the vertical axis, up to around 5 on the vertical axis) a varying speed (more in particular: movement speed of a visual stimulus) as a function of the time.

In this example, a ball (or more properly, a circle) was shown as the visual stimulus, with happy & sad faces intermittently displayed on the ball in order to alter its visual appearance, while inviting the user to press directional buttons (up corresponding with a happy face, and down corresponding with a sad face).

As can be seen from the plot, reaction times RT evolve in a manner that includes some variation, but overall the reaction times RT remain near zero on the vertical axis, meaning that the measured reaction times are sometimes faster than the allotted time duration, and sometimes slower.

Overall, the movement speed of the ball, i.e. the adaptation rate of the visual stimulus, can be seen to increase, albeit non-monotonically.

Fig. 3B, like Fig. 3A, schematically illustrates a exemplary plot of on the one hand (in the bottom region of the plot, near the horizontal axis) a varying reaction time (RT, on the vertical axis) as a function of time (on the horizontal axis), i.e. over multiple iterations of the exemplary embodiment of the method, and on the other hand (in the top region of the plot, starting from near 2 on the vertical axis, up to around 5 on the vertical axis) a varying speed (more in particular: movement speed of a visual stimulus) as a function of the time.

In this example, as in Fig. 3A, a ball was shown as the visual stimulus, but using left-pointing vs. right-pointing arrows (instead of the happy & sad faces of Fig. 3A), intermittently displayed on the ball in order to alter its visual appearance, while inviting the user to press directional buttons (of course, left corresponding with a left-pointing arrow, and right corresponding with a right-pointing arrow).

As can be seen from the plot, similar to Fig. 3A, reaction times RT evolve in a manner that includes some variation, but overall the reaction times RT remain near zero on the vertical axis, meaning that the measured reaction times are sometimes faster than the allotted time duration, and sometimes slower.

Overall, the movement speed of the ball, i.e. the adaptation rate of the visual stimulus, can be seen to increase, in this case nearly monotonically (i.e. non-decreasing, except for a single small dip).

The adaptation rate of the visual stimulus increases more quickly in Fig. 3B than in Fig. 3A, because the embodiment of Fig. 3B, because it involves changing the adaptation rate of the stimulus proportionately to a pre-determined maximum adaptation rate, and subject to a dynamic maximum adaptation rate defined as 1-(w^{T}), in which w is a weighting factor and T is the total reaction inputs. This has the effect of reaching an intended WM load more quickly (i.e. sooner).

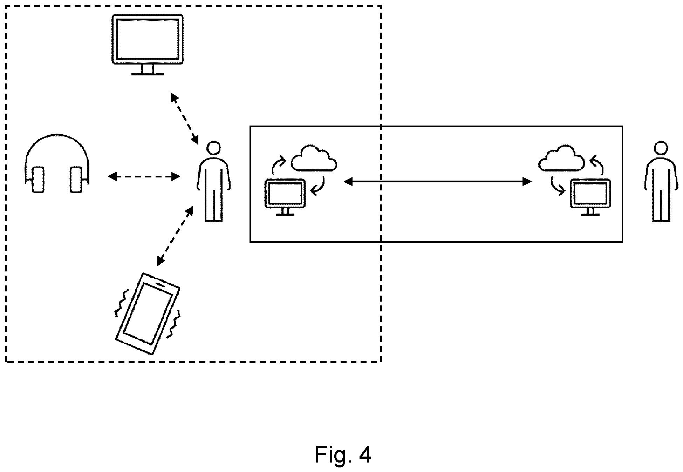

Figure 4 schematically illustrates an exemplary embodiment of the RIR testing apparatus according to the present disclosure.

The figure shows a test subject (who may also be called a client, or user) of the RIR testing apparatus on the one side, and an operator (who may also be called a therapist) of the RIR testing apparatus on the other side. Of course, the sides of the RIR testing apparatus at which the test subject and the operator are merely illustrative, and practical arrangements may vary. In some embodiments, the test subject and the operator may be situated in the same room, with a physical RIR testing apparatus. In other embodiments, the test subject may be situated remotely from the operator, and the RIR testing apparatus in the vicinity of the test subject may be configured to communicate with a control apparatus situated in the vicinity of the operator.

The RIR testing apparatus may comprise a data processing apparatus as defined hereinabove, and may comprise: at least one processor; at least one memory; at least one stimulus output interface, preferably comprising a visual display and/or a sound speaker, arranged for outputting the stimulus to a test subject; and at least one reaction input interface arranged for detecting the reaction input from the test subject in response to the stimulus. In this example, the RIR testing apparatus may further comprise a vibration unit arranged for outputting the stimulus to the test subject if the stimulus comprises a tactile stimulus.

The at least one memory may store instructions configured for, when executed by the at least one processor, controlling the RIR testing apparatus to carry out the steps of the method of any one of the above-described embodiments.

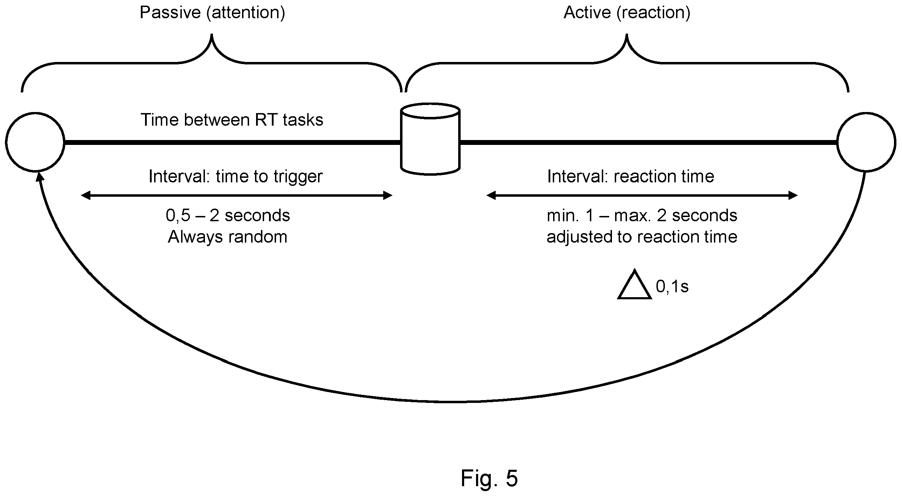

Figure 5 schematically illustrates a method operation loop of the stimulus when an exemplary embodiment of the method according to the present disclosure is performed.

The figure shows that, in this particular example, the overall method spans two phases: a passive phase focussing on attention of the test subject, and an active phase focussing on reaction of the test subject.

The figure further shows that a stimulus (in this particular example a visual stimulus, namely a round circle) can be presented, and that some time may be allowed by the control method to elapse until a trigger is activated. The elapsed time may represent an interval time up to the trigger, i.e. a time between reaction time (RT) tasks, and is preferably random between 0.5 and 2 seconds.

The figure further shows that, after the trigger has been triggered (e.g. an additional shape is shown on the stimulus), a new interval is determined, namely a reaction time (RT) interval, wherein the test subject is supposed to react. Preferably, a minimum time duration of around 1 second up to a maximum time duration of around 2 seconds is allowed to the test subject.

As is described in the present disclosure, the allowed reaction time interval (i.e. the allowable reaction time) may be increased or decreased depending on the test subject's scoring, in order to improve WM load. Preferably, the increases and/or decreases may be gradual, e.g. per 0.1 second.

The mechanism of action in Eye Movement Desensitization and Reprocessing (EMDR) therapy can be explained by the Working Memory Theory, which suggests that holding an emotional memory simultaneously while performing eye movements taxes on the limited working memory capacity. By overloading the working memory with dual attention tasks, the quality of the emotional memory is altered and the intensity weakened in reconsolidation in long term memory, leading to fewer stress-related complaints. It is also hypothesized that EMDR effectiveness increases with higher working memory load.

Scientific research has shown that making a task more difficult (e.g. faster eye movements), and adding more tasks increases working memory load.

To measure working memory load of an attention task, the Random Interval Repetition (RIR) can be used. The RIR task is a simple task used to measure working memory load via reaction time. During this task, a stimulus change is presented at alternating intervals, and patients (i.e. test subject) are required to indicate whether they have observed the stimulus change by pressing a button. Because it is simple, it is fast and sensitive, and thus capable of registering minuscule level changes of WM load. It is not currently believed to be possible to measure maximum WM load using reaction time, only relatively WM load change.

Example:
If a primary (RIR) task has a baseline mean of 500 ms, and adding a secondary task (e.g., eye movements) increases the RIR Reaction Times to 800 ms, this means that the secondary task (Eye movement) has a delaying effect on reaction time and therefore assumably increases working memory load. Higher reaction times are therefore an indication that working memory load is increasing.

A way to indicate whether working memory (over)load is reached is via the performance of an attentional task. If the patient is not able to perform the task perfectly, this is an indication that the full capacity of WM is reached. Performance can simply be measured in terms of correct/incorrect responses (errors). The more errors a patient makes, the higher the indication of working memory overload.

It is an aim of the presently-described EMDR algorithm(s) to increase the WM load of an individual to an optimal point in the shortest amount of time. This is a challenge because WM capacity and consequent level of difficulty vary among individuals. In addition, patients shouldn't be demotivated or frustrated because of a too-high level of difficulty.

### Visual RIR task

A ball moves across the screen and randomly changes within an 'interval: Time to trigger'/'time between RT tasks) into a cylinder. From this shape change (ball → cylinder), the task is 'active', and the patient must respond to this stimulus change as quickly as possible by clicking on a corresponding button. When clicked, the cylinder automatically changes back into the ball. If no click is made, the cylinder (after 'reaction time interval') automatically changes back into the ball.

The reaction times are registered by the program and have two functions:
Function 1) shortening/maintaining/prolonging the movement speed of the ball. If the reaction time is fast or slow, the speed of movement of the ball is accelerated or slowed down and the patient therefore receives a higher/lower working memory load due to the faster/slower eye movements. With a moderate reaction time changes, the interval reaction time does not change:
   - Not the speed of movement, but the ball speed is currently expressed in a representation measure of hertz: 1hz means 1 iteration (from left to right and back) within exactly 1 second. 0 is the minimum speed (0%) 1.2 Hz is the maximum speed (100%) (in the code this is shown with a different size 0 - 5.14..
   - The change of the speed of movement always happens with 'Δ0.1' (0.19%-point)
   - The starting value of movement speed = '1' (19%)
   The working memory load for the visual part (modality) of the working memory can thus be dynamically increased linearly via the ball speed with the above visual task.
Function 2) shortening/maintaining/prolonging the interval reaction time: If the reaction time is fast or slow, the interval reaction time is shortened/lengthened with the subsequent shape change (ball cylinder) and the patient therefore has less/more time to react before the cylinder automatically change back. Because the patient has less time to respond, he/she will be motivated to (continue to) respond quickly and to perform the task attentively. With a moderate reaction time, the interval reaction time does not change.
   - Fast: response within 0-20% of total interval response time.
   - Moderate: 20-40% of total interval reaction time.
   - Slow: 40%-100% of total interval reaction time.
   - The initial value interval reaction time = 2 sec.
   - The maximum interval reaction time is now set to 2 sec. and the minimum is set to 1 sec.
   - The change of the interval reaction time always happens with Δ0.1 second.

Of course, the skilled person will appreciate that the foregoing and following values are merely intended as examples, in order to help understanding of the present disclosure, and that various other values may be used instead or additionally.

### Auditory RIR task

An auditory RIR task has been developed to load the auditory part of working memory. An in-pitch ascending audio tone is presented where the patient has to click on a corresponding button as quickly as possible when he/she hears the tone. The tone continues until the patient has pressed a button or the 'interval reaction time' has passed, after which the tone automatically stops.

Function 2) shortening/maintaining/prolonging the interval reaction time: If the reaction time is fast or slow, the interval reaction time is shortened/lengthened with the subsequent shape change (ball cylinder) and the patient therefore has less/more time to react before the tone automatically stops. Because the patient has less time to respond, he/she will be motivated to (continue to) respond quickly and to perform the task attentively. With a moderate reaction time, the interval reaction time does not change.
- Fast: response within 0-20% of total interval response time.
- Moderate: 20-40% of total interval reaction time.
- Slow: 40%-100% of total interval reaction time.
- The initial value interval reaction time = 2 sec.
- The maximum interval reaction time is now set to 2 sec. and the minimum is set to 1 sec.
- The change of the interval reaction time always happens with Δ0.1 second.

### Further developed embodiments:

### Visual RIR task

A ball is presented and moves around across the screen, whereas the patient user has to follow the ball with his/her eyes. Occasionally, a visual stimulus is presented, an arrow to the left ← or an arrow to the right →, on the (moving) ball. When the arrow is presented, the task is 'active', and the patient users have to press a corresponding button (left or right arrow on the keyboard or left/right buttons on the touch-screen) as soon as they detect the arrow presented. After the arrow is presented, it disappears automatically when a button is pressed, and if not, after a time period that is based on the average reaction time + 1 Standard Deviation (function 2), making the task challenging but usually (= 69%) achievable. The interval between two consecutive tasks (time between the disappearing of an arrow and appearing of a new arrow) is random between the range of 0.5 of 2 seconds (in alignment with standard scientific practice - balancing feeling of randomness with number of available data points; also, the pauses reduce load on the working memory, which is not desirable). The reactions are recorded by the program and have the function to slow/accelerate the movement speed of the ball. If the patient's performance is good (e.g. high correct response rate), the speed of the ball is increased, and the patient, therefore, gets a higher working memory load due to using more visual cognitive resources. If the patient's performance is weak (e.g., low correct response rate, the speed of the ball is decreased, and the patient, therefore, gets a lower working memory load due to using less visual cognitive resources. The working memory load for the visual part (modality) of the working memory can thus be dynamically increased via the ball speed with the above visual task.

### Auditory RIR task

An analogy auditory task has been developed to tax the auditory part of the working memory. Occasionally, a pitch-ascending or a pitch-descending audio tone is presented. When the tone is presented, the task is 'active,' and the patient users have to press a corresponding button (up or down arrow on the keyboard or corresponding buttons on a touchscreen.) as soon as they detect the tone and whether it's ascending or descending in pitch. After the tone is presented, it disappears automatically after a time period that is based on the average reaction time + 1 Standard Deviation (function 2) or when a button is pressed within the time period. The interval between two consecutive tasks (time between the ending of a tone and the starting of a new tone) is random between the range of 0.5 of 2 seconds.

The reactions are recorded by the program and have the function to add the volume of distracting beeps. If the patient's performance is good (e.g. high correct response rate), the volume of distracting beeps is increased, and the patient, therefore, gets a higher working memory load due to more auditory stimulation. If the patient's performance is weak (e.g. low correct response rate), the volume of distracting beeps is decreased, and the patient, therefore, gets a lower working memory load due to less auditory stimulation. The working memory load for the auditory part (modality) of the working memory can thus be dynamically increased via the volume of auditory beeps with the above auditory task.

### Tactile RIR task

- It can be considered to include, additionally or alternatively, a tactile RIR task, wherein a vibration unit such as a smartphone vibrates and needs to be responded to in order for this vibration to stop.
- For all RIR tasks (visual, auditory, and tactile), the same general method is expected to work with regard to the algorithm as described above. We have to look for modal-congruent (tactile stimulation) ways to be able to load the working memory dynamically linearly (Ideas: passive distraction beeps/vibrations; varying in intrusiveness hereof; volume, strength of vibration.

As used in this application and in the claims, the singular forms "a," "an," and "the" include the plural forms unless the context clearly dictates otherwise. The systems, apparatus, and methods described herein should not be construed as limiting in any way. Instead, the present disclosure is directed toward all novel and non-obvious features and aspects of the various disclosed embodiments, alone and in various combinations and sub-combinations with one another. The disclosed systems, methods, and apparatus are not limited to any specific aspect or feature or combinations thereof, nor do the disclosed systems, methods, and apparatus require that any one or more specific advantages be present or problems be solved. Any theories of operation are to facilitate explanation, but the disclosed systems, methods, and apparatus are not limited to such theories of operation.

Although the operations of some of the disclosed methods are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed systems, methods, and apparatus can be used in conjunction with other systems, methods, and apparatus. Additionally, the description sometimes uses terms like "obtaining" and "outputting" to describe the disclosed methods. These terms are high-level abstractions of the actual operations that are performed. The actual operations that correspond to these terms will vary depending on the particular implementation and are readily discernible by the skilled person.

It will be appreciated that for simplicity and clarity of illustration, where appropriate, reference numerals may have been repeated among the different figures to indicate corresponding or analogous elements. In addition, numerous specific details are set forth in order to provide a thorough understanding of the examples described herein. However, it will be understood by the skilled person that the examples described herein can be practiced without these specific details. In other instances, methods, procedures and components have not been described in detail so as not to obscure the related relevant feature being described. The drawings are not necessarily to scale and the proportions of certain parts may be exaggerated to better illustrate details and features. The description is not to be considered as limiting the scope of the examples described herein.

Of course, the skilled person will understand that the present invention may be implemented in other ways than those specifically set forth herein without departing from the essential characteristics of the invention. The embodiments described herein are thus to be considered in all respects as illustrative and not restrictive, and all changes within the scope of the appended claims are intended to be embraced therein.

## Claims

1. A computer-implemented method of controlling a Random Interval Repetition, RIR, testing apparatus; the method comprising:
- monitoring a stimulus presented on an output interface of the RIR testing apparatus;
- monitoring a reaction input to the RIR testing apparatus in reaction to the stimulus;
- based on at least one of: a reaction time of the reaction input; and a correctness of the reaction input, changing at least one of: an adaptation rate of the stimulus; and an allowable reaction time during which a subsequent reaction to the stimulus should be input;
and repeating the method at least once.

2. The method of claim 1, wherein the adaptation rate of the stimulus comprises a visual movement speed if the stimulus comprises a visual stimulus, and/or wherein the adaptation rate of the stimulus comprises an adaptation of a ratio of background noise to stimulus signal if the stimulus comprises an auditory stimulus.

3. The method of any preceding claim, comprising: altering the stimulus, in order to elicit the reaction input in response to the alteration of the stimulus.

4. The method of the preceding claim, wherein said step of altering the stimulus is iterated over multiple iterations of the repeated method with a waiting time between iterations, the waiting time being selected randomly in a range of 0.5 to 2 seconds.

5. The method of any preceding claim, wherein said step of altering the stimulus comprises altering a visual shape of the stimulus if the stimulus comprises a visual stimulus, and/or altering a pitch tone of the stimulus if the stimulus comprises an auditory stimulus.

6. The method of any preceding claim, wherein said step of changing the adaptation rate of the stimulus based on the correctness of the reaction input comprises:
- determining, over multiple iterations of the repeated method, a ratio of correct reaction inputs to total reaction inputs; and
- changing the adaptation rate proportionately to the ratio of correct reaction inputs to total reaction inputs.

7. The method of the preceding claim, wherein said step of changing the adaptation rate of the stimulus comprises changing the adaptation rate proportionately to a pre-determined maximum adaptation rate, and subject to a dynamic maximum adaptation rate defined as 1-(w^{T}), in which w is a weighting factor and T is the total reaction inputs.

8. The method of the claim 6 or claim 7, wherein reaction inputs older than a pre-determined threshold, or reaction inputs that have been the longest in a queue threatening to overflow, are discarded.

9. The method of any preceding claim, wherein the allowable reaction time is based on an average reaction time of reaction inputs in previous iterations of the repeated method, and is preferably selected randomly within one standard deviation from said average reaction time.

10. The method of any preceding claim, wherein, if the reaction input was correct:
- the allowable reaction time, during which the subsequent reaction to the stimulus should be input, is decreased; and/or
- the adaptation rate of the stimulus is increased, preferably up to a or the pre-determined maximum adaptation rate.

11. A computer program comprising instructions which, when the program is executed by a computer, cause the computer to carry out the steps of the method of any one of claims 1-10.

12. A computer-readable storage medium comprising instructions which, when executed by a computer, cause the computer to carry out the steps of the method of any one of claims 1-10.

13. A data processing apparatus comprising means for carrying out the steps of the method of any one of claims 1-10.

14. The data processing apparatus of claim 13, forming a Random Interval Repetition, RIR, testing apparatus and comprising: at least one processor; at least one memory; at least one stimulus output interface, preferably comprising a visual display and/or a sound speaker, arranged for outputting the stimulus to a test subject; and at least one reaction input interface arranged for detecting the reaction input from the test subject in response to the stimulus; the at least one memory storing instructions configured for, when executed by the at least one processor, controlling the RIR testing apparatus to carry out the steps of the method of any one of claims 1-10.

15. The data processing apparatus of claim 14, wherein the at least one stimulus output interface comprises a vibration unit adapted to output a tactile stimulus.
